(19) Europäisches Patentamt
European Patent Office
Office européen des brevets

(11) Veröffentlichungsnummer: **0 141 076**
**A1**

(12) **EUROPÄISCHE PATENTANMELDUNG**

(21) Anmeldenummer: **84109377.6**

(22) Anmeldetag: **08.08.84**

(51) Int. Cl.⁴: **C 08 B 37/16**
A 61 K 31/00

(30) Priorität: **16.08.83 DE 3329517**

(43) Veröffentlichungstag der Anmeldung:
**15.05.85 Patentblatt 85/20**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Seidel, Heinz-Rüdiger, Dr.**
**Im Kirschfeld 15**
**D-6370 Oberursel/Taunus(DE)**

(54) **Einschlussverbindung von N,N-Dimethyl-2-chlor-5-3-methyl-2-(phenylimino)-4-thiazolin-4-ylphenylsulfonamid, Verfahren zu ihrer Herstellung sowie pharmazeutische Präparate.**

(57) Einschlußverbindung aus N,N-Dimethyl-2-chlor-5-[3-methyl-2- (phenylimino)-4-thiazolin-4-yl]phenylsulfonamid und β-Cyclodextrin, Verfahren zu ihrer Herstellung sowie pharmazeutische Präparate auf Basis dieser Verbindung. Die in vitro-Verfügbarkeit wird durch die Komplexbildung erhöht.

EP 0 141 076 A1

Croydon Printing Company Ltd.

# Einschlußverbindung von N,N-Dimethyl-2-chlor-5-[3-methyl-2-(phenylimino)-4-thiazolin-4-yl]phenylsulfonamid, Verfahren zu ihrer Herstellung sowie pharmazeutische Präparate

Die Erfindung betrifft eine Einschlußverbindung aus N,N-Dimethyl-2-chlor-5-[3-methyl-2-(phenylimino)-4-thiazolin-4-yl]phenylsulfonamid (= 4-(4-Chlor-3-dimethylsulfamoyl-phenyl)-3-methyl-2-phenylimino-4-thiazolin, HCG 497 ;vgl. Europäische Patentschrift 0023964).

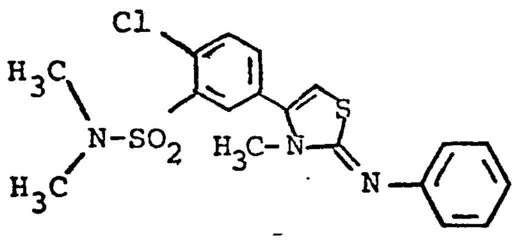

und ß-Cyclodextrin, Verfahren zu ihrer Herstellung und Zubereitungsformen auf Basis dieser Verbindung zur peroralen Verabreichung. Die Einschlußverbindung zeichnet sich durch eine hohe in vitro Verfügbarkeit aus.

HCG 497, ein Wirkstoff mit lipidsenkender Wirkung, ist schwer wasserlöslich. Er ist nur im stark sauren Medium löslich; mit zunehmendem pH-Wert nimmt seine Löslichkeit stark ab. Diese pH-abhängige Löslichkeit hat zur Folge, daß HCG 497 nur in oberen Abschnitten des Gastrointestinaltraktes gelöst und resorbiert werden kann, nicht dagegen in tieferen Abschnitten. Da die Verweilzeit in den einzelnen Abschnitten kurz ist, individuellen Schwankungen unterliegt und die im Verdauungstrakt vorherrschenden pH-Werte ebenfalls stark variieren können, ist es nicht erstaunlich, daß der Wirkstoff unter Umständen unvollständig und ungleichmäßig resorbiert wird und es zu uneinheitlicher Wirkung kommt.

Es wurde nun gefunden, daß man zur Verbesserung der Resorption die Auflösungsgeschwindigkeit des HCG 497 durch Bildung einer Einschlußverbindung mit ß-Cyclodextrin steigern kann.

Der Wirkstoff HCG 497 liegt, wie in derartigen Komplexen üblich, als Gastmolekül molekulardispers in dem Hohlraum des Cyclodextrins als Wirtsmolekül vor. Dieser Zustand stellt sozusagen das Idealergebnis einer Zerkleinerung dar, wie er durch die Herstellung fester Dispersionen oder insbesondere durch Mahlung auch nicht annähernd erreicht werden kann. Er ist für die Erhöhung der Löslichkeit und Lösungsgeschwindigkeit des eingeschlossenen Wirkstoffes verantwortlich, durch die sich Einschlußverbindungen auszeichnen.

An sich ist die Komplexbildung mit Cyclodextrin eine bekannte Reaktion. Voraussetzung dazu ist einmal, daß das Wirkstoffmolekül oder zumindest Teile davon in den Hohlraum der Cyclodextrine passen. Zum anderen muß sich der Wirkstoff in geeigneten Lösungsmitteln lösen lassen. So kann man z.B. zur Herstellung eine aetherische Lösung eines Gastmoleküls mit einer wäßrigen Cyclodextrinlösung unterschichten. An der Phasengrenze bildet sich die Einschlußverbindung; sie fällt aus (vgl. z.B. F. Cramer, F.M. Henzlein, Chem. Ber. 90, 2561(1957)).Ein anderes Verfahren besteht darin, Gastmolekül und Cyclodextrin in einer Mischung von Wasser mit wassermischbaren Lösungsmitteln wie Alkohol oder Aceton gemeinsam zu lösen und reagieren zu lassen (vgl.z.B. K.H. Frömming.,J.Weyermann, Arch. Pharmaz.305, 290 (1972)).

Zwar findet der Wirkstoff HCG 497 im ß-Cyclodextrin gut Platz. Wie sich am Kalottenmodell zeigen läßt, sind zum Einschluß 2 Moleküle Cyclodextrin erforderlich. Der Wirkstoff läßt sich aber nicht nach den beschriebenen

Herstellungsmethoden lösen und zur Reaktion bringen, so daß es aussichtslos erschien, eine Einschlußverbindung zu synthetisieren.

Es wurde nun überraschenderweise gefunden, daß man eine HCG 497-ß-Cyclodextrin-Einschlußverbindung dadurch herstellen kann, daß man aus ß-Cyclodextrin und HCG 497 unter Erwärmen eine wäßrige, saure Lösung herstellt und die Einschlußverbindung daraus isoliert. Dies war umso überraschender, als ß-Cyclodextrin bekanntlich durch Säuren gespalten wird, wobei zunächst der aus sieben Glucosemolekülen bestehende Ring des ß-Cyclodextrin aufgeht und die entstehende Kette zu Oligosacchariden abgebaut wird.

Die Erfindung betrifft also eine Einschlußverbindung aus ß-Cyclodextrin und HCG 497 sowie ein Verfahren zur Herstellung dieser Einschlußverbindung.

Zur Herstellung wird ß-Cyclodextrin zweckmäßigerweise im Überschuß eingesetzt, wobei das Molverhältnis von ß-Cyclodextrin zu HCG 497 5:1 betragen kann. Bevorzugt ist das Verhältnis 2 : 1.

Als Säuren finden anorganische und organische Säuren Verwendung; insbesondere ist Salzsäure geeignet. Der pH-Wert des abgekühlten Reaktionsgemisches beträgt etwa 1 bis 2, vorzugsweise 1,2.

Die Herstellung erfolgt z.B. in der Weise, daß aus ß-Cyclodextrin und HCG 497 in der Siedehitze eine gemeinsame wäßrige saure Lösung hergestellt wird, aus der beim Abkühlen die Einschlußverbindung ausfällt und abfiltriert wird.

Die Erfindung betrifft weiterhin pharmazeutische Zubereitungen, zu denen der Komplex verarbeitet werden kann.

Im einfachsten Fall läßt er sich in Kapseln abfüllen, eventuell nach vorausgegangener Granulation, um eine Arzneiform noch schluckbarer Größe zu erhalten. Prinzipiell ist auch eine Verabreichung des Pulvers mit und ohne Zuschlagstoffe möglich. Ferner sind auch gepreßte Formlinge geeignet Applikationsformen. Die Einschlußverbindung besitzt ebenso wie HCG 497 lipidsenkende Eigenschaften und findet daher als Arzneimittel Verwendung.

Das folgende Beispiel soll die Vorgehensweise verdeutlichen, ohne sie damit einengen oder festlegen zu wollen:

100 g (80 mMol, unter Berücksichtigung des Kristallwassergehaltes) ß-Cyclodextrin werden in 360 ml Wasser suspendiert und unter Rühren am Rückfluß bis zur Lösung erhitzt. In dieser Lösung werden 16,32 g (40 mMol) HCG 497 suspendiert. Unter weiterem Rühren und Erhitzen am Rückfluß wird 1 n-HCl (48 ml) zugetropft, bis Lösung des Wirkstoffes erfolgt. Unter Rühren läßt man nach 20 Min. abkühlen, wobei die Einschlußverbindung ausfällt. Sie wird abgenutscht, mit Wasser gewaschen und getrocknet. Man erhält ein weißes, feinkristallines Pulver in einer Ausbeute von ca. 88 g entsprechend 75 % der Theorie.

Der Gehalt an HCG 497 in den isolierten Einschlußverbindungen liegt im Bereich von 13 - 17 %, vorzugsweise von 15 - 17 %. Dies ist ein Hinweis darauf, daß die Komplexe nicht streng stöchiometrischer Zusammensetzung sind. Ein Komplex im Verhältnis von Wirkstoff zu ß-Cyclodextrin 1 : 2 würde 15,24 % HCG 497 enthalten.

Daß ein Einschluß stattgefunden hat, läßt sich mit Hilfe der Röntgenbeugung nachweisen. Reines ß-Cyclodextrin und reines HCG 497 besitzen jeweils andere Spektren als die Einschlußverbindung.

8. Verfahren zur Behandlung von Störungen des Serumlipoproteinspektrums, dadurch gekennzeichnet, daß man eine wirksame Menge einer Einschlußverbindung gemäß Anspruch 1 verabreicht.

Patentansprüche für Österreich

1. Verfahren zur Herstellung einer Einschlußverbindung aus N,N-Dimethyl-2-chlor-5[3-methyl-2-(phenylimino)-4-thiazolin-4-yl] phenylsulfonamid und ß-Cyclodextrin, dadurch gekennzeichnet, daß man aus ß-Cyclodextrin und N,N-Dimethyl-2-chlor-5-[3-methyl-2-(phenylimino)-4-thiazolin-4-yl] phenylsulfonamid unter Erwärmen eine wäßrige, saure Lösung herstellt und die Einschlußverbindung daraus isoliert.

2. Verfahren gemäß Anspruch 1, dadurch gekennzeichnet, daß man aus ß-Cyclodextrin und N,N-Dimethyl-2-chlor-5-[3-methyl-2-(phenylimino)-4-thiazolin-4-yl] phenylsulfonamid in der Hitze eine gemeinsame wäßrige, salzsaure Lösung herstellt, diese abkühlen läßt und die ausgefallene Einschlußverbindung abtrennt.

3. Verfahren gemäß Anspruch 1, dadurch gekennzeichnet, daß man ß-Cyclodextrin und N,N-Dimethyl-2-chlor-5-[3-methyl-2-(phenylimino)-4-thiazolin-4-yl] phenylsulfonamid im Verhältnis 5:1, vorzugsweise 2:1 einsetzt.

4. Verfahren zur Herstellung eines pharmazeutischen Präparates enthaltend eine Einschlußverbindung aus N,N-Dimethyl-2-chlor-5-[3-methyl-2-(phenylimino)-4-thiazolin-4-yl] phenylsulfonamid und ß-Cyclodextrin, dadurch gekennzeichnet, daß man die Einschlußverbindung in eine geeignete Applikationsform bringt.

Einen weiteren Hinweis für das Vorliegen einer Einschlußverbindung, in welcher HCG 497 in molekulardisperser Form vorliegt, gibt die Bestimmung der in vitro-Verfügbarkeit, zu deren Erhöhung die Komplexbildung durchgeführt wurde.

Füllt man zur besseren Handhabung den Komplex (erhalten gem. Beispiel) und ebenso eine 13 %ige Mischung von HCG 497 mit ß-Cyclodextrin in Kapseln und bestimmt die Wirkstoff-Freigabe im Vergleich zu Tabletten, die allein HCG 497 enthalten, erhält man folgende Ergebnisse:

| Zubereitungen mit 100 mg HCG 497 | % HCG 497, gelöst nach | |
|---|---|---|
| | 15' | 30' |
| Kapsel mit Einschlußverbindung | 48 % | 57 % |
| Kapsel mit HCG 497/ß-CyD-Mischung | 7,4 % | 13,3 % |
| Tablette | 4,8 % | 6,1 % |

Freigabemethode: Paddle: 5000 ml Puffer pH 5,5, 100 Upm

Die gefundenen Freisetzungsraten zeigen eindrucksvoll, wie durch die Überführung von HCG 497 in die erfindungsgemäße Einschlußverbindung die Auflösung gegenüber nicht behandeltem Wirkstoff um ein Vielfaches gesteigert wird.

Patentansprüche:

1. Einschlußverbindung aus N,N-Dimethyl-2-chlor-5[3-methyl-2-(phenylimino)-4-thiazolin-4-yl] phenylsulfonamid und ß-Cyclodextrin.

2. Verfahren zur Herstellung einer Einschlußverbindung gemäß Anspruch 1, dadurch gekennzeichnet, daß man aus ß-Cyclodextrin und N,N-Dimethyl-2-chlor-5-[3-methyl-2-(phenylimino)-4-thiazolin-4-yl] phenylsulfonamid unter Erwärmen eine wäßrige, saure Lösung herstellt und die Einschlußverbindung daraus isoliert.

3. Verfahren gemäß Anspruch 2, dadurch gekennzeichnet, daß man aus ß-Cyclodextrin und N,N-Dimethyl-2-chlor-5[3-methyl-2-(phenylimino)-4-thiazolin-4-yl] phenylsulfonamid in der Hitze eine gemeinsame wäßrige, salzsaure Lösung herstellt, diese abkühlen läßt und die ausgefallene Einschlußverbindung abtrennt.

4. Verfahren gemäß Anspruch 2, dadurch gekennzeichnet, daß man ß-Cyclodextrin und N,N-Dimethyl-2-chlor-5-[3-methyl-2-(phenylimino)-4-thiazolin-4-yl]phenylsulfonamid im Verhältnis 5 : 1, vorzugsweise 2 : 1 einsetzt.

5. Pharmazeutisches Präparat enthaltend eine Einschlußverbindung gemäß Anspruch 1.

6. Verfahren zur Herstellung eines pharmazeutischen Präparates, dadurch gekennzeichnet, daß man eine Einschlußverbindung gemäß Anspruch 1 in eine geeignete Applikationsform überführt.

7. Verwendung einer Einschlußverbindung gemäß Anspruch 1 zur Behandlung von Störungen des Serumlipoproteinspektrums.

Europäisches Patentamt

# EUROPÄISCHER RECHERCHENBERICHT

0141076

Nummer der Anmeldung

EP 84 10 9377

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | CHEMICAL AND PHARMACEUTICAL BULLETIN, Band 26, Nr. 4, Seiten 1162-1167; K. UEKAMA et al.: "Inclusion complexation of beta-cyclodextrin with some sulfonamides in aqueous solution" * Zusammenfassung * ----- | 1 | C 08 B 37/16<br>A 61 K 31/00 |
| | | | RECHERCHIERTE SACHGEBIETE (Int. Cl.4) |
| | | | C 08 B 37/16 |

Der vorliegende Recherchenbericht wurde für alle Patentansprüche erstellt.

| Recherchenort | Abschlußdatum der Recherche | Prüfer |
|---|---|---|
| DEN HAAG | 05-12-1984 | DE ROECK R.G. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument